# EUROPEAN PATENT APPLICATION

(11) **EP 1 688 145 A1**
(43) Date of publication of application: **09.08.2006**
(21) Application number: 06100726.6
(22) Date of filing: 23.01.2006
(51) Int. Cl.: A61K 36/324, A61K 36/76, A61K 35/64, A61P 17/06

(54) **Methods and compositions for treating psoriasis**

(30) Priority: 04.02.2005 US 649575 P
(71) Applicant: Moses, Shoshana, 2700 Kiryat-Bialik (IL)
(72) Inventor: Moses, Shoshana, 2700 Kiryat-Bialik (IL)
(74) Representative: Schmitz, Jean-Marie

(57) **Abstract**

A topical composition for treating psoriasis, comprising bee honey, Boswellia resin and willow tissue or an extract thereof and a method of using same in therapy.

## Description

### FIELD AND BACKGROUND OF THE INVENTION

The present invention relates to a method and a composition for treating psoriasis, more particularly, to utilizing a topical composition including, as active ingredients, bee honey, *Boswellia* resin and willow tissue.

Psoriasis is a chronic skin disorder in which the skin becomes inflamed, itchy, producing red and thickened scaly areas, most often on the scalp, elbows, knees, and lower back. The disorder involves epidermal hyperproliferation, disturbed keratinocyte differentiation, and inflammation of the dermis and epidermis. The exact cause of psoriasis is presently unknown.

Psoriasis is a very visible condition which has a high impact on the quality of life. It afflicts about 2% of the population and its cost of treatment is estimated to exceed $2 billion annually in the U.S.A alone.

Presently, psoriasis is treated by applying a topical agent, phototherapy, or a systemic agent. Applying a topical agent is typically the first approach to treating psoriasis. Main topical agents used for treating psoriasis are corticosteroids, anthralin and calcipotriene and tar.

The treatment of choice for providing symptomatic relief entails the topical application of corticosteroids. Corticosteroids generally have anti-inflammatory, anti-pruritic, and vasoconstrictive effects and are effective for treating itchy inflamed psoriasis. However, their long-term use is often accompanied by loss of effectiveness.

Anthralin is capable of reducing the rate of epidermal cell growth and proliferation. However, its long term use may cause irritation and staining of the skin.

Calcipotriene is a synthetic vitamin D-3 analog which regulates skin cell production. However, its long term use may cause irritation and vitamin D toxicity.

Tar has been used to treat skin conditions since ancient Egyptian times but its mode of action remains unknown. Coal tar is known to be effective in reducing inflammation and removing loose scales from patches of mild psoriasis. However, its long term use may cause stretch marks, bums and irritation.

Phototherapy is generally used only in the presence of extensive, widespread disease and following failure of treatment with topical agents. There are two main forms of phototherapy, the ultraviolet B phototherapy (UVB) which utilizes light having a wavelength in the range of 290-320 nm and the ultraviolet A phototherapy (UVA) which utilizes light having a wavelength in the range of 320-400 nm. A major drawback of associated with phototherapy is the long duration of treatment, required accessibility to specialized equipment as well as side effects such as nausea, pruritus, burning, photo damage to the skin and increased risk of skin cancer.

Systemic treatment is usually initiated only after both topical treatment and phototherapy have failed, or for patients with very active psoriatic arthritis. These are the most effective treatments for psoriasis but also have the most serious side effects. The main agents presently available are the immuno-modulators methotrexate and cyclosporine, and the oral retinoid acitretin. Adverse effects of immuno-modulators include hypertension, impaired renal function, and an increased risk of cancer, while the use of acitretin may cause dry skin and lips, sun sensitivity and an increased risk of abortion.

Hence, presently available treatments for psoriasis have limited and short-termed efficacy and/or being accompanied by serious, sometimes life threatening side effects. The present invention provides a novel topical composition for treating psoriasis which is highly effective, long-lasting, safe, devoid of side effects and simple to apply.

### SUMMARY OF THE INVENTION

According to one aspect of the present invention there is provided a topical composition for treating psoriasis, including bee honey, Boswellia resin and willow tissue or an extract thereof.

According to another aspect of the present invention there is provided an article-of-manufacturing including a packaging material and a composition identified for use in treating psoriasis being contained within the packaging material, the composition including bee honey, Boswellia resin, willow tissue or an extract thereof and a carrier suitable for topical application.

According to yet another aspect of the present invention there is provided a method of treating psoriasis. The method is effected by administering to a subject of need thereof a therapeutically effective amount of a composition including bee honey, Boswellia resin and willow tissue or an extract thereof.

According to further features in preferred embodiments of the invention described below, the bee honey is devoid of sugar additives.

According to still further features in the described preferred embodiments the willow is a species selected from the group consisting of *Salix alba, S. nigra, S. fragilis, S. purpure* and *S. babylonica.*

According to still further features in the described preferred embodiments the species is *Salix babylonica.*

According to still further features in the described preferred embodiments the willow tissue is willow bark tissue.

According to still further features in the described preferred embodiments the willow tissue is willow leaf tissue.

According to still further features in the described preferred embodiments the Boswellia is *Boswellia serrata* or *B. carterii.*

According to still further features in the described preferred embodiments the topical composition further including tar.

According to still further features in the described preferred embodiments the tar is coal tar.

According to still further features in the described preferred embodiments the tar is oil tar.

According to still further features in the described preferred embodiments the topical composition further including vinegar.

According to still further features in the described preferred embodiments the vinegar is apple cider vinegar.

According to still further features in the described preferred embodiments the topical composition further including a carrier suitable for topical application.

According to still further features in the described preferred embodiments the carrier is lard.

According to still further features in the described preferred embodiments the bee honey constitutes about 10 to 40 % of the topical composition by weight.

According to still further features in the described preferred embodiments the Boswellia resin constitutes about 10 to 40 % of the topical composition by weight.

According to still further features in the described preferred embodiments the willow tissue constitutes about 0.5 to 2 % of the topical composition by weight.

According to still further features in the described preferred embodiments the tar constitutes about 0.5 to 2 % of the topical composition by weight.

According to still further features in the described preferred embodiments the vinegar constitutes about 10 to 40 % of the topical composition by weight.

According to still further features in the described preferred embodiments the carrier constitutes about 10 to 40 % of the topical composition by weight.

According to still further features in the described preferred embodiments the subject is a human.

According to still further features in the described preferred embodiments the administering is effected by spreading and rubbing the composition onto a psoriatic tissue of the subject.

According to still further features in the described preferred embodiments the administering is effected twice daily for at least 14 days.

According to still further features in the described preferred embodiments the administering is effected twice daily for at least 28 days.

The present invention successfully addresses the shortcomings of the presently known psoriasis treatments by providing a topical composition which is highly effective for treating psoriasis, simple to apply and devoid of side effects.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the drawings:

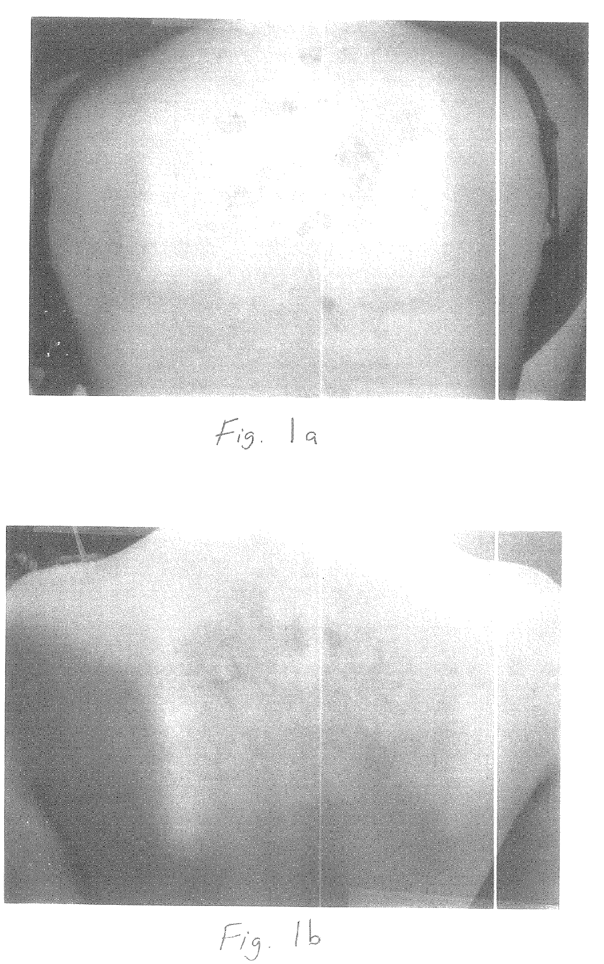

FIGs. 1a-b are photographs illustrating the effect of Formulation 3 on psoriatic patches on the back of Patient 1. Figure 1a shows the affected tissue on day 3 of treatment. Figure 1b shows the affected tissue on day 11 of treatment, indicating a marked reduction of swelling and redness of the treated patches and their splitting into multiple tiny spots encompassed by light non-pigmented skin.

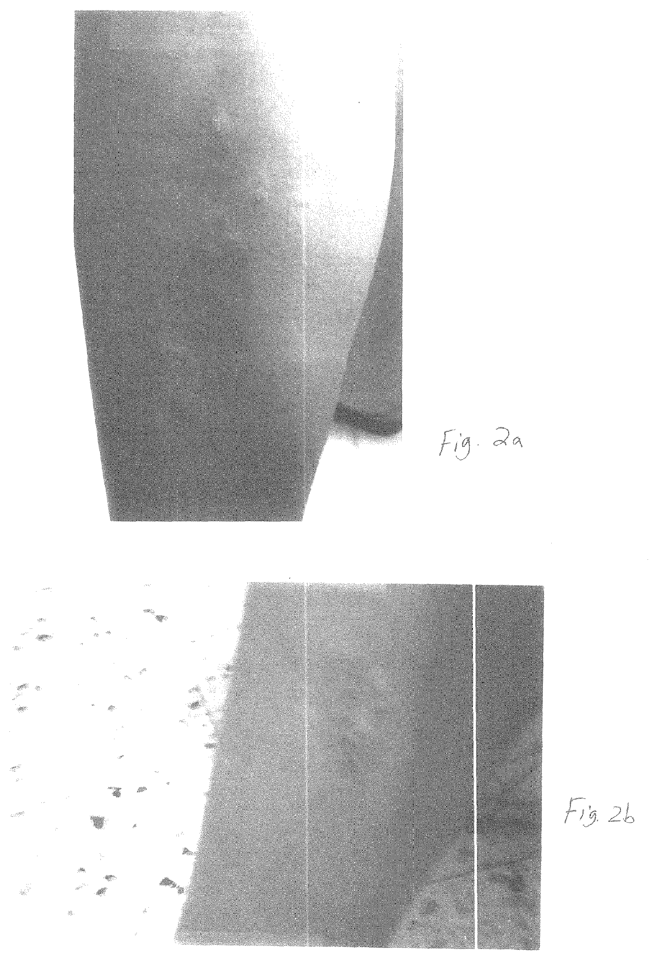

FIGs. 2a-b are photographs illustrating the effect of Formulation 3 on psoriatic patches on the foreleg of Patient 2. Figure 2a shows the affected tissue prior to treatment. Figure 2b shows an affected patch on day 10 of treatment, indicating a marked reduction of swelling and redness of the treated patch and its splitting into multiple tiny spots encompassed by light non-pigmented skin.

### DESCRIPTION OF THE PREFERRED EMBODIMENTS

The present invention is of a composition and a method of use thereof for treating psoriasis. Specifically, the composition of the present invention includes, as active ingredients, bee honey, Boswellia resin and willow tissue.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Bee honey has been known in the art as being capable of healing wounds. The use of bee honey and bee honey extracts for topically treating psoriasis is taught by U.S. Pat. No. 6,171,604.

Resins derived from Boswellia species, such as *Boswellia serrata* or *B. carterii* (commonly known as olibanum) contain boswellic acids, essential oils and triterpenoids which have anti-inflammatory activities. The use of Boswellia resin for topically treating psoriasis is taught by U.S. Pat. No. 6,589, 519 and U.S. Application Ser. No. 10/350,408.

Extracts of willow species such as *Salix alba, S. nigra. S. fragilis, S. purpure* and *S. babylonica* contain salicyn and have been used for centuries for the treatment of fever, pain (particularly low back pain), headache and inflammatory conditions such as arthritis. The use of *Salix alba* bark extract for topically treating psoriasis is taught by U.S. Pat. No. 5,165,932.

The prior art tecahes hundreds additional substances both natural and synthetic for the treatment of psoriasis. However, the prior art does not describe or suggest combining honey, Boswellia resin and willow tissue for treating psoriasis.

While reducing the present invention to practice, the present inventor surprisingly and unexpectedly uncovered that topical application of a mixture of honey, Boswellia resin and willow leaf tissue was remarkably effective in healing of psoriatic tissues. It was further observed that the treated tissue remained free of symptoms for at least five months following treatment. Supplementing the mixture with tar and vinegar further substantially accelerated the healing process (see details in Example 1 hereinbelow).

Thus according to one aspect of the present invention, there is provided a method of treating psoriasis. The method is effected by administering to a subject of need thereof a therapeutically effective amount of a composition including, as active ingredients, bee honey, Boswellia resin and willow tissue or an extract thereof.

As used herein the term "treat," "treating" or "treatment" refers to using the compositions of the present invention either to ameliorate an existing condition characterized by psoriasis or prophylactically to prevent outbreaks of psoriasis symptoms.

As used herein the term "psoriasis" refers to all skin conditions in the clinical arts described by this term, and to psoriatic-associated conditions, including psoriatic arthritis.

As used herein, the term "subject" refers to a mammal such as a human.

Preferably administering of the composition of this aspect of the present invention is effected by spreading and rubbing the composition onto a psoriatic tissue of the subject.

As used herein the phrase "psoriatic tissue" refers to skin tissue affected by psoriasis and affected cells contained within the tissue.

A suitable bee honey is preferably raw bee honey devoid of sugar additives. The present invention also envisages using a bee honey extract such as described, for example, in U.S Pat. No. 6,171,604. The concentration of bee honey in the composition of the present invention preferably constitutes about 5 to about 50%, more preferably about 10 to 40 % of the topical composition by weight.

As used herein the term "about" denotes ± 10 %. A suitable Boswellia resin can be derived from any *Boswellia* species, preferably from *B. serrata* or *B. cartenii.* Preferably the Boswellia resin is a Boswellia resin powder in its dry natural form or, alternatively, an organic solvent extract thereof. The concentration of Boswellia resin in the topical composition of the present invention preferably constitutes about 5 to 50%, more preferably about 10 to 40 % of the composition by weight.

A suitable willow species can be any *Salix* species, preferably from *Salax alba, S. nigra, S. fragilis, S. purpure* and *S. babylonica*, more preferably from *Salix babylonica*. The willow tissue of the present invention is preferably a bark tissue, a root or leaf tissue, most preferably a leaf tissue. The willow tissue is preferably an airdry tissue powder or, alternatively, an organic solvent extract thereof. The willow tissue concentration in the composition of the present invention preferably constitutes about 0.25 to about 5%, more preferably from about 0.5 to about 2 % of the composition by weight.

As can be seen in Example 1 in the Examples section which follows, the healing process induced by the composition of the present invention can be substantially enhanced by supplementing the composition with tar and vinegar. Thus, preferably, the composition of the present invention further includes tar, preferably coal tar, more preferably oil tar. The concentration of tar in the composition of the present invention preferably constitutes about 0.25 to 5%, more preferably from about 0.5 to about 2 % of the composition by weight.

The composition of the present invention may also include vinegar such as, for example, apple cider vinegar. The concentration of vinegar in the composition of the present invention preferably constitutes about 5 to 50%, more preferably about 10 to 40 % of the composition by weight.

The composition of the present invention may also include one or more additional therapeutically compatible anti-inflammatory or keratinolytic substances such as, for example, jasmine, sarsaparilla, yucca, devil's claw, feverfew, *Tagetes erecta, Moringa oleifera, Ocimum sanctum, Tridax procumbens, Aloe vera* sulfur, selenium sulfide, camphor, benzoic acid and boric acid.

A composition which constitutes the active ingredients the present invention can be used in the treatment of psoriasis *per se* or as part of a topical composition which includes at least one carrier suitable for topical application.

Hereinafter, the phrase "carrier suitable for topical application" refers to a carrier that does not cause significant irritation to a subject and does not abrogate the biological activity and properties of the administered compound. An adjuvant is included under these phrases.

A suitable carrier can be any substance which, when applied to the skin, protects and softens the skin, making it more supple. Preferably, the carrier of the present invention is an oil-based substance such as, but not limited to, an animal fat, a vegetable oil, a mineral fat, a paraffin, a wax, a moisturizing cream, an ointment, a gel, a lotion or an emulsion. More preferably, the carrier is lard.

Thus, according to one preferred embodiment of the present invention the topical composition of the present invention includes a carrier suitable for topical application, such as lard, at a concentration of, e.g., about 5 to 50%, preferably 10 to 40 % of the topical composition by weight.

Optionally, the topical composition of the present invention may include one or more excipients. The term "excipient" used herein refers to an inert substance added to a composition to further facilitate administration of an active ingredient. Examples, without limitation, of excipients include BHT, oleth-2 (CTFA), isoceteth-20 (CTFA), ascorbyl palmitate, PEG-8 (CTFA), sorbitol solution, EDTA, silicon, sodium bisulfate, emulage 100 NI, ascorbic acid, propylene glycol and sodium lauryl sulfate.

Techniques for formulation and administration of drugs may be found in "Remington's Pharmaceutical Sciences," Mack Publishing Co., Easton, PA, latest edition, which is incorporated herein by reference.

Topical compositions of the present invention may be manufactured by processes well known in the art, e.g., by means of conventional mixing, dissolving, granulating, levigating, emulsifying, encapsulating, entrapping or lyophilizing processes. The topical composition of the present invention may be formulated in various forms such as pastes, creams, ointments, lotions, gels, sticks or aerosols. Preferably, the composition is prepared by heating and continuously mixing all the ingredients in a suitable container until a uniform and homogeneous paste is formed.

Topical compositions for use in accordance with the present invention thus may be formulated in conventional manner using one or more carrier suitable for topical applications comprising excipients and auxiliaries, which facilitate processing of the active ingredients into preparations which can be used pharmaceutically.

Topical compositions suitable for use in context of the present invention include compositions wherein the active ingredients are contained in an amount effective to achieve the intended purpose. More specifically, a therapeutically effective amount means an amount of active ingredients effective to prevent, alleviate or ameliorate symptoms of a psoriasis disorder of the subject being treated.

Determination of a therapeutically effective amount is well within the capability of those skilled in the art, especially in light of the detailed disclosure provided herein.

Depending on the severity and responsiveness of the condition to be treated, dosing can be of a single or a plurality of administrations, with course of treatment lasting from several days to several weeks or diminution of the disease state is achieved. As can be seen in Example 1 of the Examples section hereinbelow, applying the preferred composition of the present invention ("Formulation 3") twice daily over a period of 28 days resulted in the complete healing of the psoriasis effected tissue. Thus, preferably, the composition of the present application is applied onto a psoriatic tissue twice daily for at least 14 consecutive days, more preferably for at least 28 consecutive days.

The amount of a composition to be administered will, of course, be dependent on the subject being treated, the severity of the affliction (e.g., area and thickness of psoriatic patches or plaques), the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

Articles-of-manufacturing comprising the topical composition of the present invention may also be prepared, placed in an appropriate container, and labeled for treatment of psoriasis, as further detailed above.

Treatment of psoriasis with the compositions of the present invention may be combined with other anti-psoriasis treatment modalities (i.e., combined therepy) which may promote therapeutic efficacy. Anti psoriasis treatment methods are described in the Background section.

Thus, the present invention provides novel compositions for treating psoriasis which are highly effective, long-lasting, non-toxic, conveniently applied, and devoid of side effects.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

### EXAMPLE 1

### Materials and Methods:

### Active ingredients:

*Bee Honey:* commercial pure bee honey devoid of sugar additives was used.

*Boswellia resin:* gum resin of *Boswellia carterii* was used.

*Willow tissue: Salix babylonica* leaves were thoroughly washed in tap water, air dried in the shed and crushed to a fine powder by a pestle and mortar.

*Tar:* industrial oil tar was used.

*Vinegar:* commercially available 100 % pure apple cider vinegar was used.

### Formulations:

*Formulation 1:* consisted of bee honey (12 ml), as an active ingredient and lard (12 ml) as a carrier.
*Formulation 2:* consisted of Formulation 1 supplemented with Boswellia resin (12 ml) and willow leaf tissue powder (0.5 gr).
*Formulation* 3: consisted of Formulation 2 supplemented with oil tar (0.5 gr) and apple cider vinegar (12 ml).

### Preparation of Topical Compositions:

For each formulation, all ingredients were provided in a stainless steel container, heated to about 90-100 °C and mixed continuously for about 5 min until a uniform and homogeneous paste was formed.

### Patients:

*Subject 1:* A 27 years old female, suffered from psoriasis since the age of 16. She has a severe psoriasis in the scalp area as well as multiple patches in the upper body area. She had been treated with extensive exposure to sunshine in the Dead Sea area over a period of three and a half years which resulted in the re-occurrence of patches, often more severely and caused her to deteriorate into an arthritic psoriasis condition. In addition, the subject had been treated with several different topical steroids and other compositions to no vale.
*Subject* 2: A 27 years old, suffered from psoriasis since the age of 12. The subject had been treated with several different topical steroids and other compositions to no vale.

### Results:

### Trial 1:

Formulation 1 was spread and rubbed by hands onto psoriatic patches twice daily for 10 consecutive days. The treatment resulted in both subjects in a substantial reduction of scaling in the treated patches within 24 hr. However, the treatment failed to reduce the psoriatic inflammation.

### Trial 2:

Formulation 2 was spread and rubbed by hands onto psoriatic patches twice daily for 45 days consecutive days. The treatment resulted in both subjects in a complete healing of the effected tissues. Following healing, the effected tissues were moisturized by lard only twice daily for one month. The effected tissues remain free of symptoms for at least five additional months.

### Trial 3:

Formulation 3 was spread and rubbed by hands onto psoriatic patches twice daily for 28 consecutive days. The treatment resulted in both subjects in an instant relief of itching. Within 10 days a marked reduction of redness associated with splitting of the treated patches into multiple tiny plaques encompassed by light skin was observed (see Figures 1a-b- 2a-b). By the end of treatment, the effected tissues were completely healed. Following healing, the effected tissues were moisturized by lard only twice daily for one month. The effected tissues remain free of symptoms for at least five additional months.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A topical composition for treating psoriasis, comprising bee honey, Boswellia resin and willow tissue or an extract thereof.

2. An article-of-manufacturing comprising a packaging material and a composition identified for use in treating psoriasis being contained within the packaging material, said composition including bee honey, Boswellia resin, willow tissue or an extract thereof and a carrier suitable for topical application.

3. A method of treating psoriasis, comprising administering to a subject of need thereof a therapeutically effective amount of a composition including bee honey, Boswellia resin and willow tissue or an extract thereof.

4. The topical composition, article-of-manufacturing and method of claim 1-3, wherein said bee honey is devoid of sugar additives.

5. The topical composition, article-of-manufacturing and method of claim 1-3, further comprising a carrier suitable for topical application.

6. The topical composition, article-of-manufacturing and method of claim 1-3, wherein said willow is a species selected from the group consisting of *Salix alba, S. nigra, S. fragilis, S. purpure* and S. *babylonica.*

7. The topical composition, article-of-manufacturing and method of claim 1-3 of claim 6, wherein said species is *Salix babylonica.*

8. The topical composition, article-of-manufacturing and method of claim 1-3, wherein said willow tissue is willow bark tissue.

9. The topical composition, article-of-manufacturing and method of claim 1-3, wherein said willow tissue is willow leaf tissue.

10. The topical composition, article-of-manufacturing and method of claim 1-3 of claim 1, wherein said Boswellia is *Boswellia serrata* or *B. carterii.*

11. The topical composition, article-of-manufacturing and method of claim 1-3, further comprising tar.

12. The topical composition, article-of-manufacturing and method of claim 11, wherein said tar is coal tar.

13. The topical composition, article-of-manufacturing and method of claim 11, wherein said tar is oil tar.

14. The topical composition, article-of-manufacturing and method of claim 1-3, further comprising vinegar.

15. The topical composition, article-of-manufacturing and method of claim 14, wherein said vinegar is apple cider vinegar.

16. The topical composition, article-of-manufacturing and method of claim 5, wherein said carrier is lard.

17. The topical composition, article-of-manufacturing and method of claim 1-3, wherein said bee honey constitutes about 10 to 40 % of the topical composition, by weight.

18. The topical composition, article-of-manufacturing and method of claim 1-3, wherein said Boswellia resin constitutes about 10 to 40 % of the topical composition by weight.

19. The topical composition, article-of-manufacturing and method of claim 1-3, wherein said willow tissue constitutes about 0.5 to 2 % of the topical composition by weight.

20. The topical composition, article-of-manufacturing and method of claim 11, wherein said tar constitutes about 0.5 to 2 % of the topical composition by weight.

21. The topical composition, article-of-manufacturing and method of claim 1-3 of claim 14, wherein said vinegar constitutes about 10 to 40 % of the topical composition by weight.

22. The topical composition, article-of-manufacturing and method of claim 5, wherein said carrier constitutes about 10 to 40 % of the topical composition by weight.
